# EUROPÄISCHE PATENTSCHRIFT

(11) **EP 1 213 022 B1**
(45) Veröffentlichungstag und Bekanntmachung des Hinweises auf die Patenterteilung: **30.07.2003**
(21) Anmeldenummer: 01129119.2
(22) Anmeldetag: 07.12.2001
(51) Int. Cl.: A61K 33/14, A61P 17/00, A61P 17/06

(54) **Verfahren zur Aufbereitung von Laist für medizinische Anwendungen, insbesondere zur Behandlung von Hautkrankheiten**
Method for purification of "Laist" for the treatment of dermatological disorders
Méthode de purification de "Laist" pour le traitement des affections dermatologiques

(30) Priorität: 08.12.2000 DE 10061232
(43) Veröffentlichungstag der Anmeldung: 12.06.2002
(73) Patentinhaber: Südsalz GmbH, 80339 München (DE)
(72) Erfinder: Patzelt, Manuela, 83451 Piding (DE); Eckert, Mathias, 83364 Neukirchen (DE); Kellerbauer, Stephan, Dr., 83487 Marktschellenberg (DE)
(74) Vertreter: Schieschke, Klaus, Dipl.-Ing.

(56) Entgegenhaltungen:
- US-A- 4 273 570
- SCHMIDT, WALTER J.: "Untersuchungen an dem zum Abdaemmen verwendeten Laist (Rueckstandston) des Ischler Salzbergwerkes" , JAHRBUCH DES OBEROESTERREICHISCHEN MUSEALVEREINES. BERICHTE, 100. PUB. DATE: 1955. P. 363. 371, ILLUS.. PUBLISHER: OBEROESTERREICHISCHER MUSEALVEREINES, LINZ, AUSTRIA. ISSN: 0379-0819 XP002189508 * Seite 366, Zeile 13-15 * * Seite 368, Zeile 1-8 *

## Beschreibung

Die Erfindung bezieht sich auf ein Verfahren zur Gewinnung und Aufbereitung von Laist für medizinische Anwendungen, insbesondere zur Behandlung von Hautkrankheiten, wie beispielsweise Psoriasis und Neurodermitis. Weiterer Bestandteil ist der vorgenannte Laist an sich, insbesondere zur Behandlung von vorgenannten Hautkrankheiten.

Ausgangspunkt sind sogenannte Aussolverfahren, welche insbesondere im Salzbergbau angewandt werden. Sie nutzen für die Gewinnung des Salzes dessen Löslichkeit in Wasser aus. Während der Verweilzeit des Wassers in einem Abbauhohlraum werden die Salze an den Hohlraumwänden aus dem Gebirge herausgelöst, während die unlöslichen Bestandteile des abgebauten Gesteins, welche als Laist bezeichnet werden, am Boden des Abbauholraumes sedimentieren.

Als Stand der Technik ist in diesem Zusammenhang ein Verfahren zur Herstellung eines Düngemittels auf Basis von Haselgebirge unter Zugabe verschiedener anderer Zusatzstoffe bekannt (US 4,273,570). Einer dieser Zusatzstoffe ist "Werkslaist", welcher jedoch nicht als alleiniger oder als wesentlicher Bestandteil des durch das bekannte Verfahren unter Schutz gestellten Düngemittels wirkt.

Es war hierbei die Aufgabe zu lösen, eine verbesserte Herstellung von Düngemitteln zu erzielen. Der Hauptbestandteil dieses Düngemittels ist in allen beschriebenen Varianten Haselgebirge mit einem variierenden Salzgehalt von 20 bis 95 % NaCl. Dieses NaCl liegt im Haselgebirge in kristalliner Form vor. Das Haselgebirge enthält hierbei praktisch keine Feuchtigkeit in Form eines Wassergehalts. Die unlöslichen Bestandteile, vorwiegend verschiedene Tonminerale und Anhydrit, bilden den im Wasser unlöslichen Rest des Haselgebirges.

Laist fand darüber hinaus in sehr geringem Umfang, beispielsweise als Mineral - Soleschlick für Gesichtsmasken, Anwendung.

Aufgabe der vorliegenden Erfindung ist es nun, ein Verfahren für die Gewinnung und Aufbereitung für Laist bzw. Laist selbst so einzusetzen, dass es als medizinisches Produkt insbesondere zur Behandlung von Hautkrankheiten verwendet werden kann.

Diese Aufgabe wird erfindungsgemäß gelöst durch Abbau von Laist in einem Abbauhohlraum, Absieben grober Bestandteile und Auswaschen der löslichen Salze, mit nachfolgenden Verfahrensschritten:
- Abbau von Laist,
- Absieben grober Bestandteile (Trockensiebung)
- Herstellen einer Suspension,
- Entwässern der Suspension,
- u.U. Kompaktieren der Suspension und Waschen,
- Trocknen,
- Mahlen des getrockneten Gutes,
- Anmischen des Mahlgutes zum Endprodukt mit einer für die Anwendung geeigneten Flüssigkeit (z.B. Wasser oder Öl) oder halbfesten Produkt (z.B. Creme, Gel, Salbe),
- Abfüllen in Verpackungseinheiten zur medizinischen Anwendung.

Das nach dem erfindungsgemäßen Verfahren hergestellte Produkt eignet sich insbesondere zur Behandlung von Psoriasis und Neurodermitis. Die Abgabe kann beispielsweise als Suspension, Paste, Creme oder Emulsion erfolgen. Es besteht auch die Möglichkeit, Laist in Form einer Packung anzuwenden, wobei hier das Endprodukt auch in bzw. auf ein Trägermaterial eingearbeitet wird.

Vorzugsweise erfolgt eine Reduzierung des Salzgehaltes des Laistes auf 1 bis 15 %.

Vorteilhafte Weiterbildungen bzw. Ausgestaltungen der Erfindung ergeben sich aus den weiteren Patentansprüchen.

Die Erfindung wird nachfolgend anhand eines Beispiels näher beschrieben:

Ausgangspunkt ist der Abbau von Laist und die Abtrennung der groben Bestandteile z.B. durch Trockensiebung, beispielsweise mittels Rüttelsieb. Anschließend erfolgt die teilweise Auswaschung der löslichen Salze durch die Herstellung einer Suspension, z.B. in einem Rührbehälter mit einem bestimmten Mischverhältnis und in einer bestimmten Lösezeit. Eine weitere Möglichkeit ist die Nasssiebung. Hierbei ist wiederum der Ausgangspunkt der Abbau von Laist. Die Absiebung erfolgt durch Nasssiebung mit einer bestimmten Flüssigkeitsmenge, beispielsweise verdünnte Sole oder Wasser, um grobe Bestandteile abzutrennen. Gleichzeitig erfolgt bereits durch diesen Verfahrensschritt ein teilweises Auswaschen löslicher Salze. Die erzeugte Suspension wird über ein geeignetes Trennaggregat, beispielsweise eine Membrankammerfilterpresse oder eine Zentrifuge, weitmöglichst entwässert. Unter Umständen wird die entwässerte Suspension kompaktiert und nochmals gewaschen, wodurch eine weitere Reduzierung des Salzgehaltes erfolgt. Die teilweise entsalzte und weitmöglichst entwässerte Suspension wird getrocknet. Dies erfolgt beispielsweise durch Konvektionstrocknung mittels Heißluft oder Kontakttrocknung im Vakuum. Das getrocknete Gut wird anschließend zerkleinert. Dies erfolgt beispielsweise in Kugelmühlen, Schwingmühlen oder Luftstrahlmühlen. Die Trennung der Fraktionen erfolgt durch geeignete Trennaggregate, beispielsweise Windsichter oder Siebmaschinen. Eine weitere Möglichkeit ist die gleichzeitige Trocknung und Mahlung der teilweise entsalzten und weitmöglichst entwässerten Suspension durch Mahltrocknung.

Das erhaltene Mahlgut wird zum Endprodukt mit einer für die Anwendung geeigneten Flüssigkeit, insbesondere Wasser, Gelbildner, Öl und/oder weitere pharmazeutische Stoffe, oder halbfesten Produkt angemischt. Dies erfolgt beispielsweise durch einen Chargenmischer oder einen kontinuierlichen Mischprozess. Anschließend wird das Endprodukt in Packungseinheiten abgefüllt, wie beispielweise Dosen, Tuben oder Folien.

Der für das erfindungsgemäße verfahren verwendete Laist weist durchschnittliche folgende in Wasser unlösliche Hauptbestandteile, bezogen auf Trockensubstanz auf:

| | | |
|---|---|---|
| SiO₂ | (Siliziumdioxid) | 45 - 60 % |
| Al₂O₃ | (Aluminiumoxid) | 10 - 25 % |
| CaO | (Calziumoxid) | 0 - 1 % |
| MgO | (Magnesiumoxid) | 3 - 15 % |
| Fe₂O₃ | (Eisen-II-oxid) | 5 - 10 % |
| K₂O | (Kaliumoxid) | 2 - 8 %. |

Im Laist sind im Wesentlichen folgende Mineralien enthalten. Die Angaben beziehen sich auf die Trockensubstanz:

| | |
|---|---|
| Muskovit/Illit | 20 - 30 % |
| Quarz | 20 - 25 % |
| Chlorit | 15 - 25 % |
| lösl. Salze (NaCl+KCl) | 10 - 15 % |
| Hämatit | 0 - 5 % |
| Kaolinit | 0 - 5 % |
| Magnesit | 0 - 2 % |
| Anhydrit | 1 - 10 % |
| Calcit | 0 - 2% |
| Apatit | 0 - 0,5 % |
| Gips | 1 - 5 % |
| Polyhalit | 0 - 1,5 %. |

Das damit hergestellte Endprodukt dient zur medizinischen Anwendung, insbesondere zur Behandlung von Hautkrankheiten. Es kann beispielsweise als Suspension, Paste, Creme, Emulsion oder als Packung angewendet werden.

Ausgangsmaterial ist hierbei ein Rohstoff, nämlich Laist, welcher beim Aussolverfahren in einem Abbauhohlraum eines Salzbergwerkes entsteht. Damit ergibt sich ein medizinisch sehr effektives Produkt.

## Patentansprüche

1. Verfahren zur Gewinnung und Aufbereitung von Laist für medizinische Anwendungen, insbesondere zur Behandlung von Hautkrankheiten (Psoriasis, Neurodermitis), durch Abbau von Laist aus einem Sinkwerk und Auswaschen der löslichen Salze, mit folgenden Verfahrensschritten
a) Herstellen einer Suspension,
b) Entziehen der flüssigen Phase,
c) Trocknen,
d) Mahlen des getrockneten Gutes,
e) Anmischen des Mahlgutes zum Endprodukt,
f) Abfüllen des Endproduktes in Verpackungseinheiten.

2. Verfahren nach Anspruch 1, **gekennzeichnet durch** Entfernen bzw. Abtrennen grober Bestandteile vor Herstellen der Suspension.

3. Verfahren nach Anspruch 2, **gekennzeichnet durch** Absieben der groben Bestandteile **durch** Trockensiebung oder Nasssiebung, Herstellen einer Suspension in einem Rührbehälter.

4. Verfahren nach Anspruch 1 oder 2, **gekennzeichnet durch** Kompaktieren der Suspension und Waschen zur weitergehenden Entsalzung.

5. Verfahren nach Anspruch 1, **gekennzeichnet durch** thermische oder Vakuumtrocknung.

6. Verfahren nach einem oder mehreren der vorhergehenden Ansprüche, **gekennzeichnet durch** Anmischen des Mahlgutes zum Endprodukt mit einer für die Anwendung vorgesehenen Flüssigkeit, insbesondere Wasser, Gelbildner, Öl und/oder weitere pharmazeutische Stoffe, oder in Form eines halbfesten Produktes.

7. Verfahren nach einem oder mehreren der vorhergehenden Ansprüche, **gekennzeichnet durch** Reduzierung des Salzgehaltes im Endprodukt auf 1-15%.

8. Verwendung des nach den vorhergehenden Ansprüchen hergestellten Endproduktes, insbesondere zur Behandlung von Hautproblemen, in Form von auf der Haut auftragbaren Suspensionen, Cremes, Emulsionen oder Packungen.

9. Laist zur medizinischen Anwendung, insbesondere zur Behandlung von Hautkrankheiten nach einem oder mehreren der vorhergehenden Ansprüche.

## Claims

1. Method for obtaining and preparing laist for medicinal applications, in particular for the treatment of skin diseases (psoriasis, neuro-dermatitis), by working laist from a sink works and washing out the soluble salts, the method having the following method steps:
a) production of a suspension,
b) extraction of the liquid phase,
c) drying,
d) grinding the dried material,
e) mixing the ground material to form the end product,
f) decanting the end product into packing units.

2. Method according to claim 1, **characterised by** removal or separation of coarse constituents before the production of the suspension.

3. Method according to claim 2, **characterised by** sieving off the coarse constituents by dry-sieving or wet-sieving, production of a suspension in a stirrer vessel.

4. Method according to claim 1 or 2, **characterised by** compacting the suspension and washing for the purposes of more extensive desalination.

5. Method according to claim 1, **characterised by** thermal or vacuum drying.

6. Method according to one or more of the preceding claims, **characterised by** mixing the ground material, to form the end product, with a liquid that is provided for the application, in particular water, gelling agent, oil and/or further pharmaceutical substances, or in the form of a semi-solid product.

7. Method according to one or more of the preceding claims, **characterised by** reduction of the salt content in the end product to 1-15%.

8. Use of the end product produced according to the preceding claims, in particular for the treatment of skin problems, in the form of suspensions, creams, emulsions or packs that can be applied to the skin.

9. Laist for medicinal application, in particular for the treatment of skin diseases according to one or more of the preceding claims.

## Revendications

1. Méthode d'extraction et de préparation de résidus de lessivage pour des applications médicales, en particulier pour le traitement des affections dermatologiques (psoriasis, neurodermatoses) par exploitation du résidus de lessivage provenant d'une exploitation par dissolution, et lessivage des sels solubles, comprenant les étapes suivantes :
a) fabrication d'une suspension,
b) extraction de la phase liquide,
c) séchage,
d) broyage du produit sec,
e) mélange du produit sec pour obtenir le produit fini,
f) remplissage d'unités de conditionnement avec le produit fini.

2. Procédé selon la revendication 1, **caractérisé par** l'enlèvement et/ou la séparation des gros composants avant fabrication de la suspension.

3. Procédé selon la revendication 2, **caractérisé par** un tamisage des gros composants par tamisage à sec ou tamisage humide, la fabrication d'une suspension dans un mélangeur-brasseur.

4. Procédé selon les revendications 1 ou 2, **caractérisé par** un compactage de la suspension et par un lavage pour poursuivre le dessalement.

5. Procédé selon la revendication 1, **caractérisé par** un séchage thermique ou sous vide.

6. Procédé selon l'une quelconque des revendications précédentes, **caractérisé en ce qu'**on mélange la matière moulue en vue d'obtention du produit fini à un liquide prévu pour l'application, en particulier de l'eau, des gélifiants, de l'huile et/ou d'autres substances pharmaceutiques, ou sous la forme d'un produit semi-solide.

7. Procédé selon une ou plusieurs des revendications précédentes, **caractérisé par** une réduction à 1-1,5 % de la teneur en sel du produit fini.

8. Utilisation du produit fini fabriqué selon les revendications précédentes, en particulier pour le traitement de problèmes de peau, sous la forme de suspensions, crèmes, émulsions ou enveloppements applicables sur la peau.

9. Résidus de lessivage pour une application médicale, en particulier pour le traitement des maladies de la peau, selon une ou plusieurs des revendications précédentes.
